# NOUVEAU FASCICULE DE BREVET EUROPEEN

(11) **EP 0 685 222 B2**
(45) Date de publication et mention de la décision concernant l'opposition: **21.06.2000**
(45) Mention de la délivrance du brevet: 11.09.1996
(21) Numéro de dépôt: 95401056.7
(22) Date de dépôt: 05.05.1995
(51) Int. Cl.: A61K 7/42

(54) **Compositions cosmétiques photoprotectrices et utilisations**
Kosmetische Sonnenschutzmittel und Verwendungen
Cosmetic sunscreening compositions and uses

(30) Priorité: 03.06.1994 FR 9406830
(43) Date de publication de la demande: 06.12.1995
(73) Titulaire: L'OREAL, 75008 Paris (FR)
(72) Inventeur: Hansenne, Isabelle, F-75017 Paris (FR); van Leeuwen, Alexandra Victoria, F-75020 Paris (FR)

(56) Documents cités:
- EP-A- 0 087 098
- EP-A- 0 365 370
- EP-A- 0 521 651
- WO-A-94/04131
- FR-A- 2 680 105
- GB-A- 2 198 944
- Teach-in "Several aspects in formulating UV-Absorbers" in Cosmetics and Toiletries from R. Langer (Haarman & Reimer GmbH)
- Seifen-Öle-Fette-Wachse 106, nr. 7/1980, p. 199-200
- Compositions and dates of first manufecturing of the Johnson & Johnson products "Sundown broadspectrum maximum protection sunscreen","Johnson's baby sunblock lotion and creme" and "Sundown creme"
- Römpp Chemie Lexikon, 9th Ed., 1990, pp. 2537-2540
- Cosmetics & Toiletries Sun Products, vol. 105, Dec. 1990, pp. 122-124, 75 and 72
- Bernel Chemical Company Inc, Three Suntan Oils, SPF 15, 19 & 20
- "US federal register", vol. 58, nr. 90, May 12, 1993, Proposed rules p. 28295-28296
- Heinz A Staab, Einführung in die Theoretische Organische Chemie, 4. Auflage, 1964, p. 665
- Christian Reichardt, Lösungsmittel-Effekte in der organischen Chemie, 1969, p. 5
- Positivliste der Kosmetikverordnung, Anlage 7, 02/37, mai 1996 + 02/38, april 1995
- Techsnische Information "Eusolex" UV-Strahlenfilter für Kosmetika, von Merck, gedruckt und verteilt seit 8.7.1999
- Déclaration d'Isabelle Hansenne reçue le 16.6.1999
- Solubility and related properties, Kenneth C. James, Marcel Dekker, INC, 1986, pp. 1-7, 36-47

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), ainsi que leur utilisation dans l'application cosmétique susmentionnée. Plus précisément encore, elle concerne des compositions antisolaires comprenant, dans un support cosmétiquement acceptable, une association d'au moins trois filtres lipophiles particuliers, l'un des filtres possédant des propriétés solubilisantes vis à vis des autres filtres. L'invention concerne par ailleurs un procédé de solubilisation de deux filtres lipophiles solides particuliers au moyen d'un ou deux autres filtres lipophiles liquides spécifiques.

On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un-vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions cosmétiques destinées à la photoprotection (UV-A et/ou UV-B) de la peau ont été proposées à ce jour.

Pour diverses raisons liées en particulier à un meilleur confort d'utilisation (douceur, émollience, facilité d'application et autres), les compositions antisolaires actuelles se présentent le plus souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques capables d'absorber selectivement les rayonnements UV nocifs, ces filtres étant sélectionnés en fonction de l'indice de protection recherché (l'indice de protection (IP) s'exprimant mathématiquement par le rapport du temps d'irradiation nécessaire pour atteindre le seuil érythématogène avec le filtre UV au temps nécessaire pour atteindre le seuil érythématogène sans filtre UV). Selon leur caractère lipophile ou au contraire hydrophile, ces filtres peuvent se répartir, respectivement, soit dans la phase grasse, soit dans la phase aqueuse, de la composition finale.

Il s'avère que deux filtres particulièrement intéressants et largement utilisés à ce jour sont constitués d'une part par le 4-méthylbenzylidène camphre, qui est notamment vendu sous la dénomination commerciale de "EUSOLEX 6300" par la Société Merck, et, d'autre part, par le 4-(ter.-butyl) 4'-méthoxy dibenzoylméthane, qui est, quant à lui, notamment proposé à la vente sous la dénomination commerciale de "PARSOL 1789" par la Société GIVAUDAN.

Il s'agit de deux filtres lipophiles, fortement actif dans l'UV-B pour le premier et fortement actif dans l'UV-A pour le second, qui présentent la particularité mais aussi le désavantage d'être tous deux solides à température ambiante. De ce fait, leur utilisation, en combinaison, dans une composition cosmétique antisolaire implique certaines contraintes au niveau de leur formulation et de leur mise en oeuvre, en particulier lorsqu'il s'agit de trouver des solvants permettant de les solubiliser correctement. A cet égard, on fait aujourd'hui le plus souvent appel à des huiles comme des esters et plus particulièrement des benzoates d'alkyles en C₁₂-C₁₅ ("FINSOLV TN" de chez Finetex), ou à des triglycérides et notamment des triglycérides d'acides gras en C₈-C₁₂ ("MIGLYOL 812" de chez Hüls), ou encore à des monoalcools ou des polyols tels que l'éthanol, ainsi qu'à leurs mélanges. Ces produits, bien que possédant des propriétés solubilisantes vis à vis des deux filtres susmentionnés, présentent néanmoins pour inconvénient de n'avoir aucune activité propre dans le domaine de la filtration du rayonnement UV, tant UV-A qu'UV-B.

Or, la Demanderesse vient maintenant de découvrir, de façon inattendue et surprenante, que le salicylate d'homomenthyle (encore appelé plus simplement homosalate) et le salicylate d'octyle constituent tous deux, pris seuls ou en mélange, un solvant particulièrement remarquable pour les deux filtres solides susmentionnés, à savoir le 4-méthylbenzylidène camphre et le 4-(ter.-butyl) 4'-méthoxy dibenzoylméthane, ces derniers composés présentant en effet dans ces premiers des solubilités extrèmement élevées, et en tous cas nettement supérieures à celles obtenues avec tous les autres solvants usuels utilisés à ce jour, ce qui permet, à quantité égale de solvant, de mettre en oeuvre des quantités plus importantes de filtres. On notera que les salicylates d'homomenthyle d'une part et d'octyle d'autre part sont des filtres lipophiles liquides déjà connus pour leur activité dans l'UV-B. L'intérêt de la présente découverte est ainsi double, en ce sens qu'il est maintenant possible d'une part d'effectuer la solubilisation du 4-méthylbenzylidène camphre et du 4-(ter.-butyl) 4'-méthoxy dibenzoylméthane, par un solvant autre que ceux antérieurement connus, ce qui est toujours intéressant en soi, et ceci, d'autre part, tout en réalisant une augmentation substantielle, à concentration égale de ces deux filtres dans la composition antisolaire finale, du niveau de protection attachée à cette dernière.

Toutes ces découvertes sont à la base de la présente invention.

Ainsi, conformément à l'un des objets de la présente invention, il est maintenant proposé de nouvelles compositions cosmétiques, en particulier antisolaires, qui sont caractérisées par le fait qu'elles comprennent, dans un support cosmétiquement acceptable, (i) un système filtrant solubilisé constitué d'un mélange entre le 4-méthylbenzylidène camphre et le 4-(ter.-butyl) 4'-méthoxy dibenzoylméthane, et (ii) un système filtrant solubilisant dont les constituants sont choisis parmi le salicylate d'homomenthyle, le salicylate d'octyle et leurs mélanges, ledit système filtrant solubilisant étant présent en une quantité suffisante pour solubiliser à lui seul l'ensemble dudit système filtrant solubilisé.

La présente invention a également pour objet l'utilisation des compositions ci-dessus comme, ou pour la fabrication de, compositions cosmétiques destinées à la protection de la peau et/ou des cheveux contre le rayonnement ultraviolet, en particulier le rayonnement solaire.

Un autre objet encore de la présente invention réside dans un procédé de traitement cosmétique pour la protection de la peau et/ou des cheveux contre le rayonnement ultraviolet, en particulier le rayonnement solaire, et qui consiste essentiellement à appliquer sur ces derniers une quantité efficace d'une composition conforme à l'invention.

La présente invention a également enfin pour objet l'utilisation du salicylate d'homomenthyle et/ou du salicylate d'octyle pour solubiliser du 4-méthylbenzylidène camphre et du 4-(ter.-butyl) 4'-méthoxy dibenzoylméthane contenus dans une composition cosmétique antisolaire.

D'autres caractéristiques, aspects et avantages de la présente invention apparaitront à la lecture de la description détaillée qui va suivre.

Comme indiqué précédemment, le 4-méthylbenzylidène camphre (composé A destiné à être solubilisé) est un filtre connu en soi, actif dans l'UV-B et se présentant sous une forme solide, qui est vendu notamment sous la dénomination commerciale de "EUSOLEX 6300" par la Société MERCK ou de "PARSOL 5000" par la Société GIVAUDAN. Ce produit répond à la formule (I) suivante :

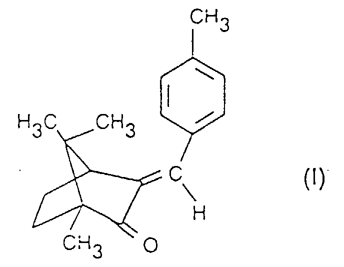

De même, le 4-(ter.-butyl) 4'-méthoxy dibenzoylméthane (composé B destiné à être solubilisé), qui est un filtre solide actif dans l'UV-A connu en soi, est, pour sa part, notamment proposé à la vente sous la dénomination commerciale de "PARSOL 1789" par la Société GIVAUDAN, et il répond à la formule (II) suivante :

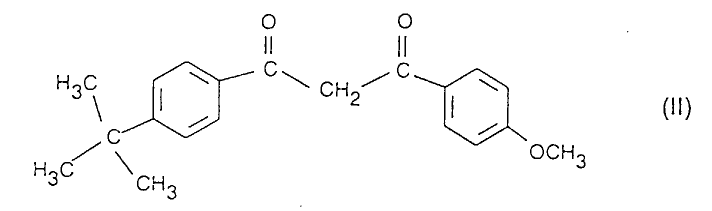

Concernant le salicylate d'homomenthyle (composé C destiné à solubiliser les composés A et/ou B), connu aussi sous le nom d'homosalate, il s'agit également d'un produit disponible commercialement, et il est vendu notamment sous la dénomination de "KEMESTER HMS" par la Société Witco. Il répond à la formule (III) suivante :

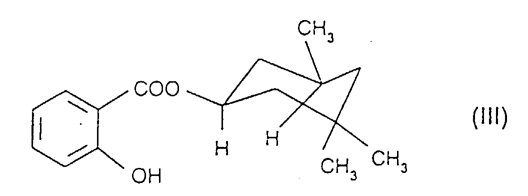

Enfin, le salicylate d'octyle (composé D destiné à solubiliser, conjointement ou non avec le composé solubilisant C, les composés A et/ou B) est, quant à lui, vendu notamment sous la dénomination commerciale de "UVINUL O-18" par la Société BASF, et il répond à la formule (IV) suivante :

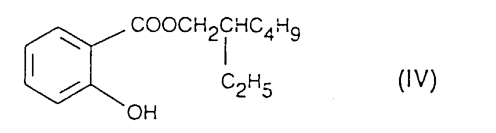

Les composés A et B (filtres à solubiliser) peuvent être présents dans les compositions selon l'invention à une concentration comprise entre 0,25 et 15 % en poids par rapport au poids total de la composition. Selon une caractéristique essentielle de la présente invention, ces composés doivent se présenter dans la composition finale sous une forme totalement, ou substantiellement totalement, solubilisée.

Les composés C et/ou D (solubilisants) peuvent être, quant à eux, présents dans les compositions selon l'invention à des teneurs comprises entre 0,5 et 20 % en poids par rapport au poids total de la composition. Selon une caractéristique essentielle des compositions selon l'invention, ces composés doivent être utilisés, seuls ou en mélanges, en une quantité telle qu'elle soit suffisante pour solubiliser à elle seule la totalité, ou substantiellement la totalité, du composé A et du composé B [(composé A) + (composé B)] présent dans la composition. Cette quantité minimale en solvant(s) destinée à assurer une dissolution complète et stable du ou des filtres solides peut être classiquement déterminée à partir de l'étude des paramètres de solubilité desdits filtres dans ces solvants.

A titre indicatif, il a été constaté que, à température ambiante, le composé A est soluble à raison de 40 % en poids dans le composé solvant C, et à raison de 50 % en poids dans le composé solvant D ; le composé B est, quant à lui, soluble à raison de 50 % en poids dans le composé solvant C, et à raison de 30 % en poids dans le composé solvant D ; enfin, un mélange contenant un composé A et un composé B dans un rapport pondéral 3:1 est soluble à raison de 55 % en poids dans le composé solvant C, et à raison de 57 % en poids dans le composé solvant D.

D'une manière générale, on notera que les concentrations en composés A et/ou B et en composés C et/ou D, sont choisies de manière telle que l'indice de protection solaire de la composition finale soit de préférence d'au moins 2.

Selon une caractéristique particulièrement avantageuse des compositions conformes à l'invention, ces dernières ne contiennent de préférence aucun, ou substantiellement aucun, solubilisant pour les composés (A+B) autre que le ou les composés C et D précédemment définis. Selon l'invention, on considère qu'un composé donné ne possède pas de propriétés solubilisantes vis à vis d'un autre composé donné lorsque ce dernier composé présente une solubilité inférieure à 1 % en poids environ dans ce premier composé.

En outre, toujours selon un mode préféré de réalisation de la présente invention, le support cosmétiquement acceptable dans lequel se trouvent contenus les différents composés A, B, C ou D, est une émulsion de type huile-dans-eau.

Le système filtrant à l'état solubilisé conforme à l'invention est constitué par l'association des deux filtres lipophiles solides susmentionnés, à savoir un mélange entre le filtre UV-B qui est ici le 4-méthylbenzylidène camphre et le filtre UV-A qui est ici le 4-(ter.-butyl) 4'-méthoxy dibenzoylméthane, car on peut ainsi disposer finalement de formulations antisolaires qui offrent une protection maximale dans toute la gamme des UV nocifs (280 nm - 400 nm), ces dernières compositions présentant par ailleurs la propriété d'être parfaitement stables.

Les compositions cosmétiques antisolaires selon l'invention peuvent bien entendu contenir un ou plusieurs filtres solaires complémentaires actifs dans l'UVA et/ou l'UVB (absorbeurs), hydrophiles ou lipophiles autres, bien sûr, que tous les filtres lipophiles mentionnés ci-avant. Ces filtres complémentaires peuvent être notamment choisis parmi les dérivés cinnamiques, les dérivés salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflection et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les opacifiants, les stabilisants, les émollients, les silicones, les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges, et ils comprennent également les acides gras, les alcools gras et les esters d'acides gras. Les huiles peuvent être choisies parmi les huiles animales, végétales, minérales ou de synthèse et notamment parmi l'huile de vaseline, l'huile de paraffine, les huiles de silicone, volatiles ou non, les isoparaffines, les poly-α-oléfines, les huiles fluorées et perfluorées. De même, les cires peuvent être choisies parmi les cires animales, fossiles, végétales, minérales ou de synthèse connues en soi.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Les compositions de l'invention peuvent être préparées selon les techniques bien connues de l'homme de l'art, en particulier celles-destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse où de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse représente généralement de 50 à 95 % en poids, de préférence de 70 à 90 % en poids, par rapport à l'ensemble de la formulation, la phase huileuse (comprenant les filtres lipophiles solubilisés et solubilisants) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10% en poids, par rapport à l'ensemble de la formulation. On notera que, conformément à la présente invention, la phase grasse de telles émulsions peut n'être constituée pour l'essentiel ou même en totalité que des composés C et/ou D (filtres organiques solvants) dans lesquels se trouvent solubilisés les filtres A et B précédemment définis, ainsi que les éventuels filtres complémentaires et autres adjuvants cosmétiques lipophiles classiques.

Comme indiqué en début de description, un autre objet de la présente invention réside dans un procédé de traitement cosmétique de la peau ou des cheveux destiné à les protéger contre les effets des rayons UV consistant à appliquer sur ceux-ci une quantité efficace d'une composition cosmétique telle que définie ci-dessus.

Des exemples concrets, mais nullement limitatifs, illustrant l'invention, vont maintenant être donnés.

### EXEMPLE 1

### Emulsion huile-dans-eau :

- 4-méthylbenzylidène camphre ("EUSOLEX 6300") 6g
- 4-(ter.-butyl) 4'-méthoxy dibenzoylméthane ("PARSOL 1789") 2 g
- Salicylate d'homomenthyle 10 g
- TiO2 de qualité nanopigmentaire 4 g
- Mélange d'alcool cétylstéarylique et d'alcool cétylstéarylique oxyéthyléné à 33 moles d'oxyde d'éthylène vendu sous la dénomination "Sinnowax AO" par HENKEL (émulsionnant) 7 g
- Mélange de mono- di et tristéarate de glycérol (co-émulsionnant) 2 g
- Triglycérides d'acides gras en C8-C12 ("MIGLYOL 812") 3 g
- Polydiméthylsiloxane 1,5 g
- Alcool cétylique 1,5 g
- Conservateurs qs
- Eau distillée qsp 100g

On réalise l'émulsion ci-dessus en dissolvant les filtres dans la phase grasse, puis en ajoutant les (co)émulsionnants dans cette phase grasse portée aux environs de 80°C, et enfin en additionnant sous agitation rapide l'eau préalablement chauffée à cette même température.

### EXEMPLE 2

### Emulsion huile-dans-eau :

- 4-méthylbenzylidène camphre ("EUSOLEX 6300") 3g
- 4-(ter.-butyl) 4'-méthoxy dibenzoylméthane ("PARSOL 1789") 1 g
- Salicylate d'octyle 8 g
- Acide benzène 1,4 [di(3-méthylidènecampho-10-sulfonique)] 1,5 g
- Mélange d'alcool cétylstéarylique et d'alcool cétylstéarylique oxyéthyléné à 33 moles d'oxyde d'éthylène vendu sous la dénomination "Sinnowax AO" par HENKEL (émulsionnant) 7 g
- Mélange de mono- di et tristéarate de glycérol (co-émulsionnant) 2 g
- Triglycérides d'acides gras en C8-C12 ("MIGLYOL 812") 3 g
- Polydiméthylsiloxane 1,5 g
- Alcool cétylique 1,5 g
- Conservateurs qs
- Eau distillée qsp 100g

Cette émulsion a été préparée comme à l'exemple 1.

### EXEMPLE 3

### Emulsion eau-dans-huile :

- 4-méthylbenzylidène camphre ("EUSOLEX 6300") 6 g
- 4-(ter.-butyl) 4'-méthoxy dibenzoylméthane ("PARSOL 1789") 2 g
- Salicylate d'octyle 8 g
- Salicylate d'homomenthyle 5 g
- Mélange [hydroxystéarate et isostéarate de sorbitol et de glycérol] à 20 moles d'oxyde de propylène et 30 moles d'oxydes d'éthylène, vendu sous le nom de "ARLACEL 780" par ICI 2,5 g
- Conservateurs qs
- Parfum qs
- Eau qsp 100g

## Revendications

1. Compositions cosmétiques à usage topique, en particulier pour la photoprotection de la peau et/ou des cheveux, caractérisées par le fait qu'elles comprennent, dans un support cosmétiquement acceptable, (i) un système filtrant solubilisé, constitué d'un mélange entre le 4-méthylbenzylidène camphre (composé A) et le 4-(ter.-butyl) 4'-méthoxy dibenzoylméthane (composé B) et (ii) un système filtrant solubilisant dont les constituants sont choisis parmi le salicylate d'homomenthyle (composé C), le salicylate d'octyle (composé D) et leurs mélanges, ledit système filtrant solubilisant étant présent en une quantité suffisante pour solubiliser à lui seul l'ensemble dudit système filtrant solubilisé.

2. Compositions selon la revendication 1, caractérisées par le fait que le ledit système filtrant solubilisé est présent à raison de 0,25 à 15 % en poids par rapport à l'ensemble de la composition.

3. Compositions selon l'une des revendications 1 ou 2, caractérisées par le fait que ledit système filtrant solubilisant est présent à raison de 0,5 à 20 % en poids par rapport à l'ensemble de la composition.

4. Compositions selon l'une quelconque des revendications précédentes, caractérisées par le fait qu'elles sont exemptes ou substantiellement exemptes d'un agent solubilisant pour les composés (A+B) autre que le ou les composés C et D.

5. Compositions selon l'une quelconque des revendications précédentes, caractérisées par le fait que ledit support cosmétiquement acceptable se présente sous la forme d'une émulsion de type huile-dans-eau.

6. Compositions selon l'une quelconque des revendications précédentes, caractérisées par le fait qu'elles comprennent en outre un ou plusieurs filtres organiques complémentaires actifs dans l'UV-A et/ou UV-B, hydrophiles ou lipophiles.

7. Compositions selon la revendication 6, caractérisées par le fait que lesdits filtres organiques complémetaires sont choisis parmi les dérivés cinnamiques, les dérivées salicyliques, les dérivés du camphre, les dérivés de triazine, les dérivés de la benzophénone, les dérivés du dibenzoylméthane, les dérivés de β,β-diphénylacrylate, les dérivés de l'acide p-aminobenzoïque, les polymères filtres et les silicones filtres.

8. Compositions selon l'une quelconque des revendications précédentes, caractérisées par le fait qu'elles comprennent en outre, à titre d'agents photoprotecteurs complémentaires, des pigments ou nanopigments d'oxydes métalliques, enrobés ou non, capables de bloquer physiquement, par diffusion et/ou réflection, le rayonnement UV.

9. Compositions selon la revendication 8, caractérisées par le fait que lesdits pigments ou nanopigments sont choisis parmi les oxydes de titane, de zinc, de fer, de zirconium, de cérium et leurs mélanges, enrobés ou non.

10. Compositions selon l'une quelconque des revendications précédentes, caractérisées par le fait qu'elles comprennent en outre au moins un agent de bronzage et/ou de brunissage artificiel de la peau.

11. Compositions selon l'une quelconque des revendications précédentes, caractérisées par le fait qu'elles comprennent en outre au moins un adjuvant choisi parmi les corps gras, les solvants organiques, les épaississants ioniques ou non ioniques, les adoucissants, les antioxydants, les opacifiants, les stabilisants, les émollients, les silicones, les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants,, les colorants.

12. Compositions selon l'une quelconque des revendications précédentes, caractérisées par le fait qu'il s'agit de compositions protectrices de l'épiderme humain ou de compositions antisolaires et qu'elles se présentent sous forme de dispersions vésiculaires non ioniques, émulsions, en particulier émulsions de type huile-dans-eau, crèmes, laits, gels, gels crèmes, suspensions, dispersions, poudres, bâtonnets solides, mousses ou spray.

13. Compositions selon l'une quelconque des revendications 1 à 11, caractérisées par le fait qu'il s'agit de compositions de maquillage des cils, des sourcils ou de la peau et qu'elles se présentent sous forme solide ou pâteuse, anhydre ou aqueuse, d'émulsions, de suspensions ou de dispersions.

14. Compositions selon l'une quelconque des revendications 1 à 11, caractérisées par le fait qu'il s'agit de compositions destinées à la protection des cheuveux contre les rayons ultraviolets et qu'elles se présentent sous forme de shampooings, de lotions, de gels, d'émulsions, de dispersions vésiculaires non ioniques ou de laques pour cheveux.

15. Compositions selon l'une quelconque des revendications précédentes, caractérisées par le fait qu'elles présentent un indice de protection sur peau d'au moins 2.

16. Utilisation des compositions définies à l'une quelconque des revendications précédentes comme, ou pour la fabrication de, compositions cosmétiques pour la protection de la peau et/ou des cheveux contre le rayonnement ultraviolet, en particulier le rayonnement solaire.

17. Procédé de traitement cosmétique pour protéger la peau et/ou les cheveux contre le rayonnement ultraviolet, en particulier le rayonnement solaire, caracterisé en ce qu'il consiste à appliquer sur ceux-ci une quantité efficace d'une composition telle que définie à l'une quelconque des revendications 1 à 15.

18. Utilisation d'un système constitué de salicylate d'homomenthyle et/ou de salicylate d'octyle en une quantité suffisante pour solubiliser à lui seul l'ensemble du 4-méthylbenzylidène camphre et du 4-(ter.-butyl) 4'-méthoxy dibenzoylméthane contenus dans une composition cosmétique antisolaire.

## Claims

1. Cosmetic compositions for topical use, in particular for the photoprotection of the skin and/or the hair, characterized in that they comprise, in a cosmetically acceptable support, (i) a solubilized screening system consisting of a mixture of 4-methylbenzylidenecamphor (compound A) and 4-(tert-butyl)-4'-methoxydibenzoylmethane (compound B), and (ii) a solubilizing screening system whose constituents are chosen from homomenthyl salicylate (compound C), octyl salicylate (compound D) and mixtures thereof, the said solubilizing screening system being present in an amount which is sufficient to solubilize by itself all of the said solubilized screening system.

2. Compositions according to Claim 1, characterized in that the said solubilized screening system is present in an amount of from 0.25 to 15 % by weight relative to the whole composition.

3. Compositions according to either of Claims 1 or 2, characterized in that the said solubilizing screening system is present in an amount of from 0.5 to 20 % by weight relative to the whole composition.

4. Compositions according to any one of the preceding claims, characterized in that they are free or substantially free of a solubilizing agent for the compounds (A + B) other than the compound or compounds C and D.

5. Compositions according to any one of the preceding claims, characterized in that the said cosmetically acceptable support is in the form of an emulsion of oil-in-water type.

6. Compositions according to any one of the preceding claims, characterized in that they additionally comprise one or more additional hydrophilic or lipophilic organic screening agents active in the UV-A and/or UV-B range.

7. Compositions according to Claim 6, characterized in that the said additional organic screening agents are chosen from cinnamic derivatives, salicylic derivatives, camphor derivatives, triazine derivatives, benzophenone derivatives, dibenzoylmethane derivatives, β,β-diphenylacrylate derivatives, p-aminobenzoic acid derivatives, screening polymers and screening silicones.

8. Compositions according to any one of the preceding claims, characterized in that they additionally comprise, as additional photoprotective agents, coated or noncoated metal oxide pigments or nanopigments which are capable of physically blocking, by diffusion and/or reflection, UV radiation.

9. Compositions according to Claim 8, characterized in that the said pigments or nanopigments are chosen from titanium oxide, zinc oxide, iron oxide, zirconium oxide, cerium oxide and mixtures thereof, which may or may not be coated,

10. Compositions according to any one of the preceding claims, characterized in that they additionally comprise at least one agent for the artificial tanning and/or browning of the skin.

11. Compositions according to any one of the preceding claims, characterized in that they additionally comprise at least one adjuvant chosen from fats, organic solvents, ionic or nonionic thickening agents, softeners, antioxidants, opacifying agents, stabilizing agents, emollients, silicones, α-hydroxy acids, anti-foaming agents, hydrating agents, vitamins, fragrances, preserving agents, surfactants, fillers, sequestering agents, polymers, propellants, basifying or acidifying agents and dyes.

12. Compositions according to any one of the preceding claims, characterized in that they are compositions for protecting the human epidermis or antisun compositions and in that they are in the form of nonionic vesicle dispersions, emulsions, in particular emulsions of oil-in-water type, creams, milks, gels, cream gels, suspensions, dispersions, powders, solid sticks, foams or spray.

13. Compositions according to any one of Claims 1 to 11, characterized in that they are compositions for making up the eyelashes, the eyebrows or the skin and in that they are in anhydrous or aqueous, solid or pasty form, in emulsion form, suspension form or dispersion form.

14. Compositions according to any one of Claims 1 to 11, characterized in that they are compositions intended for protection of the hair against ultraviolet rays and in that they are in the form of shampoos, lotions, gels, emulsions, nonionic vesicle dispersions or lacquers for the hair.

15. Compositions according to any one of the preceding claims, characterized in that they have a protection factor on skin of at least 2.

16. Use of the compositions defined in any one of the preceding claims as, or for the manufacture of, cosmetic compositions for the protection of the skin and/or the hair against ultraviolet radiation, in particular solar radiation.

17. Cosmetic treatment process for protecting the skin and/or the hair against ultraviolet radiation, in particular solar radiation, characterized in that it consists in applying an effective amount of a composition as defined in any one of Claims 1 to 15 to the skin and/or the hair.

18. Use of a system consisting of homomenthyl salicylate and/or of octyl salicylate in an amount sufficient to solubilize by itself all of the 4-methylbenzylidenecamphor and the 4-(tert-butyl)-4'-methoxydibenzoylmethane contained in an antisun cosmetic composition.

## Patentansprüche

1. Kosmetische Zusammensetzungen zur topischen Anwendung, insbesondere zum Lichtschutz der Haut und/oder der Haare,
dadurch gekennzeichnet, daß
sie in einem kosmetisch akzeptablen Träger (i) ein solubilisiertes Filtersystem, das aus einem Gemisch von 4-Methylbenzylidencampher (Verbindung A) und 4-(tert.-Butyl)-4'-methoxydibenzoylmethan (Verbindung B) besteht, und (ii) ein solubilisierendes Filtersystem, dessen Bestandteile unter Homomenthylsalicylat(Verbindung C), Octylsalicylat (Verbindung D) und deren Gemischen ausgewählt sind, enthalten, wobei das solubilisierende Filtersystem in einer Menge enthalten ist, die allein ausreicht, das gesamte solubilisierte Filtersystem zu solubilisieren.

2. Zusammensetzungen nach Anspruch 1, dadurch gekennzeichnet, daß das solubilisierte Filtersystem in einem Mengenanteil von 0,25 bis 15 Gew.-%, bezogen auf die gesamte Zusammensetzung, enthalten ist.

3. Zusammensetzungen nach einem der Ansprüche 1 oder 2, dadurch gekennzeichnet, daß das solubilisierende Filtersystem in einem Mengenanteil von 0,5 bis 20 Gew.-%, bezogen auf die gesamte Zusammensetzung, enthalten ist.

4. Zusammensetzungen nach einem der vorhergehenden Ansprüche, dadurch gekennzeichnet, daß sie frei oder im wesentlichen frei von solubilisierenden Mitteln für die Verbindungen (A+B) sind, die von der oder den Verbindungen C und D verschieden sind.

5. Zusammensetzungen nach einem der vorhergehenden Ansprüche, dadurch gekennzeichnet, daß der kosmetisch akzeptable Träger in Form einer Öl-in-Wasser-Emulsion vorliegt.

6. Zusammensetzungen nach einem der vorhergehenden Ansprüche, dadurch gekennzeichnet, daß sie ferner ein oder mehrere ergänzende hydrophile oder lipophile organische Filter, die im UV-A-Bereich und/oder UV-B-Bereich wirksam sind, enthalten.

7. Zusammensetzungen nach Anspruch 6, dadurch gekennzeichnet, daß die ergänzenden organischen Filter unter den Zimtsäurederivaten, Salicylsäurederivaten, Campherderivaten, Triazinderivaten, Benzophenonderivaten, Dibenzoylmethanderivaten, β,β-Diphenylacrylatderivaten, p-Aminobenzoesäurederivaten, polymeren Filtern und Siliconfiltern ausgewählt werden.

8. Zusammensetzungen nach einem der vorhergehenden Ansprüche, dadurch gekennzeichnet, daß sie als ergänzende Lichtschutzmittel ferner gegebenenfalls umhüllte Pigmente oder Nanopigmente von Metalloxiden enthalten, die die UV-Strahlung physikalisch durch Streuung und/oder Reflexion zu sperren vermögen.

9. Zusammensetzungen nach Anspruch 8, dadurch gekennzeichnet, daß die Pigmente und Nanopigmente unter gegebenenfalls umhüllten Oxiden von Titan, Zink, Eisen, Zirconium, Cer und deren Gemischen ausgewählt sind.

10. Zusammensetzungen nach einem der vorhergehenden Ansprüche, dadurch gekennzeichnet, daß sie außerdem mindestens ein Bräunungsmittel und/oder ein Mittel zur künstlichen Hautbräunung enthalten.

11. Zusammensetzungen nach einem der vorhergehenden Ansprüche, dadurch gekennzeichnet, daß sie ferner mindestens einen Zusatzstoff enthalten, der unter den Fettsubstanzen, organischen Lösungsmitteln, ionischen oder nichtionischen Verdikkungsmitteln, Mitteln für die Geschmeidigkeit, Antioxidantien, Trübungsmitteln, Stabilisatoren, Emollentien, Siliconen, α-Hydroxysäuren, Antischaummitteln, Hydratisierungsmitteln, Vitaminen, Parfums, Konservierungsmitteln, grenzflächenaktiven Stoffen, Füllstoffen, Maskierungsmitteln, Polymeren, Treibmitteln, alkalisch oder sauer machenden Mitteln und Färbemitteln ausgewählt sind.

12. Zusammensetzungen nach einem der vorhergehenden Ansprüche, dadurch gekennzeichnet, daß es sich um Zusammensetzungen, die die menschliche Epidermis schützen, oder Sonnenschutzmittel handelt und daß sie in Form von nichtionischen Vesikeldispersionen, in Form von Emulsionen, insbesondere Öl-in-Wasser-Emulsionen, Cremes, Milchen, Gelen, Gelcremes, Suspensionen, Dispersionen, Pudern, festen Stiften, Schäumen und Sprays vorliegen.

13. Zusammensetzungen nach einem der Ansprüche 1 bis 11, dadurch gekennzeichnet, daß es sich um Zusammensetzungen zum Schminken der Wimpern, Augenbrauen oder der Haut handelt und daß sie in fester oder pastöser, wasserfreier oder wäßriger Form, als Emulsionen, Suspensionen oder Dispersionen vorliegen.

14. Zusammensetzungen nach einem der Ansprüche 1 bis 11, dadurch gekennzeichnet, daß es sich um Zusammensetzungen zum Schutz der Haare gegen UV-Strahlung handelt und daß sie in Form von Haarwaschmittel, Lotion, Gel, Emulsion, nichtionischer Vesikeldispersion oder Haarlack vorliegen.

15. Zusammensetzungen nach einem der vorhergehenden Ansprüche, dadurch gekennzeichnet, daß sie einen Lichtschutzfaktor auf der Haut von mindestens 2 aufweisen.

16. Verwendung von Zusammensetzungen nach einem der vorhergehenden Ansprüche als kosmetische Zusammensetzungen oder für die Herstellung von kosmetischen Zusammensetzungen zum Schutz der Haut und/oder der Haare gegen UV-Strahlung und insbesondere gegen Sonnenlicht.

17. Verfahren zur kosmetischen Behandlung zum Schutz der Haut und/oder der Haare gegen UV-Strahlung und insbesondere Sonnenlicht, dadurch gekennzeichnet, daß eine wirksame Menge einer in den Ansprüchen 1 bis 15 definierten Zusammensetzung auf die Haut aufgebracht wird.

18. Verwendung eines Systems, das aus Homomenthylsalicylat und/oder Octylsalicylat besteht, in einer Menge, die allein ausreicht, um die gesamten, in einer kosmetischen Sonnenschutzzusammensetzung enthaltenen Verbindungen 4-Methylbenzylidencampher und 4-(*tert*.-Butyl)-4'-methoxydibenzoylmethan zu solubilisieren.
